# EUROPEAN PATENT SPECIFICATION

(11) **EP 0 518 277 B1**
(45) Date of publication and mention of the grant of the patent: **09.09.1998**
(21) Application number: 92109722.6
(22) Date of filing: 10.06.1992
(51) Int. Cl.: C12N 15/05, C12N 1/02

(54) **Method for separating and culturing fused hybrid cells**
Verfahren zur Trennung und Kultivierung von fusionierten Hybridzellen
Procédé de séparation et de culture de cellules hybrides fusionnées

(30) Priority: 11.06.1991 JP 235315/91
(43) Date of publication of application: 16.12.1992
(73) Proprietor: Japan Tobacco Inc., Shinagawa-ku, Tokyo 140 (JP)
(72) Inventor: Kasaoka, Keisuke, c/o Japan Tobacco Inc., Toyoda-cho, Iwata-gun, Shizuoka 438 (JP); Kadotani, Naoto, c/o Japan Tobacco Inc., Toyoda-cho, Iwata-gun, Shizuoka 438 (JP); Matsushima, Hideko, c/o Japan Tobacco Inc., Toyoda-cho, Iwata-gun, Shizuoka 438 (JP)
(74) Representative: Reinhard, Horst, Dr.

(56) References cited:
- BIOLOGICAL ABSTRACTS, vol. 85, 1988, Philadelphia, PA (US); Y. KAMATA et al., AN 68263

## Description

### SPECIFICATION

### BACKGROUND OF THE INVENTION

### I. Field of the Invention

This invention relates to a method for separating and culturing fused hybrid cells in a plant cell suspension after cell fusion treatment.

### II. Description of the Related Art

In the cells in a plant cell suspension obtained after cell fusion treatment, the percentage of the fused hybrid cells is at best about 5%, so that the remaining 95% of the cells in the cell suspension (i.e., the cells which are not fused hybrid cells) need not be cultured in the subsequent step.

A method for efficiently selecting the fused hybrid cells in such a plant cell suspension has been proposed in which mesophyll protoplasts and epidermal cells are fused, and the fused hybrid cells are separated by centrifuging the cell suspension under a density gradient of parcoal ("Theoretical and Applied Genetics" Vol. 75, 1987, pp. 26 - 29), utilizing the differences in the specific gravities therebetween. However, this method has a drawback in that obtaining epidermal cells is laborious and time-consuming.

A method in which protoplasts treated with iodoacetamide (IOA) which cannot undergo cell division by themselves are used as a parent has also been proposed ("Tissue Culture", Vol. 15 (11), 1989, p. 344 - 348). However, with this method, since the protoplasts which are the other parent, as well as fused cells between these protoplasts, can divide by themselves, the survived cells contains these cells which are not fused hybrid cells, so that it is impossible to select only thee fused hybrid cells.

Still another method has been proposed, in which chlorotic plants are prepared by treating a plant with norflurazon and chlorotic protoplasts prepared from the chlorotic plant are used as a parent, and the fused hybrid cells are separated by using a micromanipulator or a cell sorter ("Plant Tissue Culture Association of Japan", Abstract of First Plant Tissue Culture Colloquium, 1988, pp. 24 - 27; and "Plant Science", Vol. 56, 1988, pp. 61 - 68. However, with this method, selecting the fused hybrid cells using a micromanipulator under microscope needs high skill and is laborious, and the cell sorter is expensive and its operation also requires high skill.

### SUMMARY OF THE INVENTION

Accordingly, an object of the present invention is to provide a method for separating and culturing fused hybrid cells, by which substantially fused hybrid cells alone are separated after the cell fusion by a simple operation that does not require skillfulness.

The present inventors intensively studied to find that by mixing mesophyll protoplasts treated with iodoacetamide and chlorotic mesophyll protoplasts originating from a plant treated with norflurazon, and subjecting the mixture to cell fusion treatment; centrifuging the obtained cell suspension; and by culturing the cells contained in the fraction precipitated by the centrifugation, substantially fused hybrid cells alone may be separated, thereby completing the present invention.

That is, the present invention provides a method for separating and culturing fused hybrid cells comprising, in the order mentioned, the steps of mixing mesophyll protoplasts which cannot divide unless they are fused with a cell having an ability to undergo cell division and chlorotic mesophyll protoplasts originating from a plant treated with norflurazon, and subjecting the mixture to cell fusion treatment; centrifuging the obtained cell suspension; and culturing the cells contained in the fraction precipitated by the centrifugation.

In the method of the present invention, the selection of the fused hybrid cells is carried out by centrifugation utilizing the fact that the specific gravity of the chlorotic mesophyll protoplast obtained by the treatment with norflurazon is smaller than that of the normal green mesophyll protoplast. The method of the present invention is clearly distinguished in this point from the conventional method in which norflurazon-treated chlorotic protoplasts are used. That is, in the conventional method, the fused hybrid cells are selected by observing the cells with a microscope and selecting the fused hybrid cells by using a micromanipulator, or by selecting the fused hybrid cells by using a cell sorter. Thus, in the conventional method employing the norflurazon-treated chlorotic mesophyll protoplasts, the selection is not made by centrifugation utilizing the differences in specific gravities. In the present invention, since the selection is carried out by centrifugation, skill is not required for the selection and the method is suited for treating a large number of cells.

It is also a characteristic feature of the present invention that the protoplasts to be fused with the above-described chlorotic protoplasts cannot divide unless they are fused with a cell having an ability to divide. By employing such protoplasts as one of the parents and the above-described chlorotic protoplasts as the other parent, the efficiency of the selection is drastically promoted when compared with the conventional methods.

The reason is as follows: In the conventional method, the protoplasts to be fused with the IOA-treated protoplasts having no cell-dividing ability are normal protoplasts which did not undergo a special treatment. Thus, in the cell suspension after the cell fusion. treatment, the cells which do not start cell division are only the IOA-treated mesophyll protoplasts which were not fused and the fused cells formed by the cell fusion between the IOA-treated mesophyll protoplasts. Therefore, not only the desired fused hybrid cells formed by the cell fusion between the IOA-treated protoplast and the normal protoplast, but also the normal protoplasts and fused cells formed by the cell fusion between the normal protoplasts start cell division. Thus, it is impossible to select only the desired fused hybrid cells.

In contrast, in the method of the present invention, the protoplasts to be fused with the protoplasts having no cell-dividing ability are chlorotic protoplasts. Therefore, by the centrifugation of the cell suspension after the cell fusion treatment, the non-fused chlorotic protoplasts and the fused cells formed by the cell fusion between the chlorotic protoplasts can be eliminated because their specific gravities are smaller than those of the green protoplasts and the desired fused hybrid cells. Thus, in the precipitated fraction obtained by the centrifugation, substantially all of those which can divide are the desired fused hybrid cells.

By the above-described mechanism, the selection efficiency of the fused cells is drastically promoted.

Thus, with the method of the present invention, the selection step which is carried out for increasing the percentage of the desired fused hybrid cells in the cells cultured in the subsequent step as high as possible can be carried out without skill, and considerably large number of cells can be treated in one batch. Further, the step of culturing the selected cells to obtain regenerated plants was also drastically improved. Therefore, since a large number of regenerated plants originating from the desired fused hybrid cells may be obtained, the development of new plants will be drastically promoted.

### DETAILED DESCRIPTION OF THE PREFERRED EMBODIMENTS

As mentioned above, in the method of the present invention, mesophyll protoplasts which cannot divide unless they are fused with a cell having an ability to under go cell division (hereinafter also referred to as "protoplasts having no cell-dividing ability") and chlorotic mesophyll protoplasts are mixed and the mixture is subjected to a cell fusion treatment.

The protoplasts having no cell-dividing ability are known in the art and any of these protoplasts may be employed. The protoplasts having no cell-dividing ability may be obtained by treating protoplasts with an cell-inactivating agent such as IOA, iodoacetate and diethylpyrrocarbonate. More particularly, the IOA-treated mesophyll protoplasts may be obtained by, for example, immersing mesophyll protoplasts originating from a green leaf, which are obtained by a conventional method, in an aqueous IOA solution with a concentration of 5 - 30 mM at a low temperature (preferably 0 - 10°C) for 5 - 30 minutes, and washing the resultant. The protoplasts may preferably be washed with aqueous mannitol solution with a concentration of preferably 0.3 - 0.6 M. Similarly, the protoplasts may be treated with iodoacetate solution or with diethylpyrocarbonate solution.

The above-described mesophyll protoplasts having no cell-dividing ability do not divide by themselves and can undergo cell division only when they are fused with the chlorotic protoplasts which will now be described in more detail.

On the other hand, the chlorotic mesophyll protoplasts may be prepared, for example, as follows:

A seedling of a plant is transplanted to a culture medium containing 10 - 20 mg/l of norflurazon and the plant is cultured to obtain a chlorotic plant. In order to obtain chlorotic mesophyll protoplasts having high cell-dividing abilities, it is preferred to culture the seedling firstly in a medium containing no norflurazon, and norflurazon is then added to the medium during the culture, thereby obtaining a plant whose upper portion alone is chlorotic.

Then the mesophyll of the thus obtained chlorotic plant is cut into rectangular pieces with a width of 0.5 - 2 mm. The method for preparing protoplasts are wellknown in the art and is described in, for example, H. Binding et al., Physiol. Plant, Vol. 43, 1977, pp.52-54.

Then the above-described two kinds of protoplasts are mixed and the mixture is subjected to a cell fusion treatment. Any conventional cell fusion treatment may be employed. The electrofusion method, however, is best preferred. In this case, a cell suspension with a prescribed cell population is prepared for each kind of protoplasts and the cell suspensions are mixed. The cell population of the cell suspension may preferably be 10⁴ - 10⁵ cells/ml. The mixed suspension is placed in an apparatus for electrofusion and subjected to electrofusion. The conditions of electrofusion may be, for example, as follows:
Frequency of high frequency wave: 0.5 - 2 MHz
Voltage of high frequency wave: 50 - 200 V/cm
Time for applying voltage: 1 - 20 seconds
Pulse voltage: 1 - 2 KV/cm
Pulse width: 10 - 20 microseconds
Interval between pulses: 0.2 - 1.0 second
Number of pulses applied: 2 - 10 times
It should be noted that the conditions of electrofusion are not restricted to those just mentioned above. The electrofusion method is described in, for example, V. Zimmermann, P. Scheurich, G. Pilawat and R. Benz, Angew-Chem., Int. ed., 20, 325 (1981).

The cell suspension after the cell fusion treatment is placed in a test tube and the fraction precipitated by the centrifugation is collected. Although not restricted, the centrifugation may preferably be carried out under an acceleration of 7 - 15 G for 3 - 5 minutes. To increase the purity, it is preferred to wash the precipitate with a washing solution and carry out the centrifugation under the above-mentioned conditions. As the washing solution, aqueous mannitol solution with a concentration of 0.3 - 0.6 M is preferred. This operation may preferably be repeated several times.

By the centrifugation, the fused hybrid cells which are heavy because of the chloroplasts contained therein and which have abilities to divide are precipitated, while the chlorotic protoplasts as well as the fused cells between the chlorotic protoplasts remain in the supernatant since they are light in weight because they do not contain chloroplasts. Although the protoplasts having no cell-dividing ability contain chloroplasts, since they cannot divide by themselves, they are screened out during the subsequent culturing.

Then the precipitate is transferred to a medium and the fused hybrid cells are cultured according to a conventional method. The culturing may be carried out in a known medium suited for the fused hybrid cells according to a conventional method. Examples of the culture medium which may be used here is described in, for example, J.F. Shepard and R.E. Totter, Plant Physiol., Vol. 60, 1977, pp. 313 - 316, although the medium is not restricted to those described in this reference. A suitable culturing temperature may be selected for the fused hybrid cells to be cultured, which may usually be 15 - 25^{o}C.

The fused hybrid cells in the precipitate may preferably be embedded in agarose gel and may be cultured therein. This may be carried out by, for example, as follows: A culture medium is added to a cell suspension prepared by suspending the cells in the precipitate so as to appropriately adjust the cell population. Then agarose solution is added to the medium and the resulting mixture is stirred while keeping the mixture at a temperature at which agarose is not solidified. Then the mixture is allowed to cool so as to solidify agarose. The agarose gel containing the cells is then placed under culturing conditions. The cell population may preferably be 10⁴ - 10⁵ cells/ml and the concentration of the agarose solution may preferably be 0.5 - 1.0% by weight.

Even more preferably, the cells are cultured in agarose gel drops. This may be carried out by, for example, as follows:

To a cell suspension prepared by suspending the cells contained in the precipitate, a culture medium is added so as to adjust the cell population to 10⁴ - 10⁵ cells/ml. On the other hand, a culture medium to which agarose is added is prepared, and this culture medium is kept at a temperature at which the agarose is not solidified (e.g., 40°C). These two culture media are mixed and uniformly stirred. The resulting mixture is dropped on several points in a petri dish and the drops are then allowed to solidify. The volume of each drop may preferably be 5 - 100 µl. Then a culture medium is placed in the petri dish to a level such that the tops of the drops are covered. The petri dish is then placed in an incubator and the cells are cultured therein.

By employing the culturing method in which the cells are embedded in agarose gel or in agarose gel drops, the percentage of the cells which initiate cell division is increased, so that the plants regenerated from the fused hybrid cells may be efficiently obtained.

The present invention will now be described by way of examples thereof. It should be noted that the examples are presented for the illustration purpose only and should not be interpreted in any restrictive way.

### Examples

### Treatment for Obtaining Chlorotic Mesophyll Protoplasts

Chlorotic mesophyll protoplasts were obtained from potatoes (variety: May Queen and Konafubuki) by the following method: Tetraploids of the potatoes were cultivated in a green house and when they flowered, they were pollinated with pollen of Solanum phureja so as to induce parthenocarpy. In the obtained seeds, those having no embryo spots were aseptically cultured in LS medium (Linsmaier & Skoog medium). Aseptic seedlings having no violet bands in the nodes were selected to obtain dihaploids of the potatoes.

In 300 ml plant boxes, LSP5 liquid medium having the composition shown in Table 1 below was placed in an amount of 20 ml per each plant box, and the medium was sterilized. To the liquid medium, single node or several nodes obtained from the above-described aseptic seedlings of dihaploid of the potatoes were placed, and cultured at 20°C for 2 weeks in an incubator whose inside is illuminated (5,000 lux) for 18 hours a day.

**Table 1**

| Composition of LSP5 Medium | |
|---|---|
| Component | Concentration (g/l) |
| LS Medium* | |
| myo-Inositol | 0.1 |
| Thiamine HCl | 0.001 |
| Sucrose (pH 5.6) | 5.0 |

| | |
|---|---|
| *: Linsmaier and Skoog medium (1965) | |

When the plantlets were grown to 5 - 10 leaf stage, norflurazon was added to the liquid medium to a concentration of 10 ppm. The potatoes were cultured in the same conditions for another 2 weeks. As a results, potatoes whose upper portions alone are chlorotic were obtained.

The mesophyll of the chlorotic leaves in the upper portions of the potatoes were cut into pieces with widths of 1 - 2 mm, and the resulting pieces were immersed in an enzyme solution POT-9 for isolating protoplasts at 25^{o}C for 16 hours, which has the composition shown in Table 2 below.

**Table 2**

| Composition of Enzyme Solution POT-9 | |
|---|---|
| Component | Concentration (g/l) |
| Meicelase (P-1)* | 5.0 |
| Pectriase (Y-23)** | 0.5 |
| 2-(N-Morpholino)ethanesulfonic | 1.0 |
| Acid (MES) | |
| Polyvinylpyrrolidone | 20.0 |
| (PVP 40) | |
| Mannitol (pH 5.6) | 91.0 |

| | |
|---|---|
| *: commercially available from Meiji Seika Kaisha, Ltd. | |
| **: commercially available from Morishin Seiyaku Co., Ltd. | |

### Treatment of Mesophyll Protoplasts with IOA

Mesophyll protoplasts to be treated with IOA were obtained from Solanum brevidens (diploid) which is a wild type potato, dihaploid lines of May Queen (tetraploid) which is a cultivated variety of potato, Momotaro which is a cultivated variety of tomato, and LS-89 which is a variety for stock of tomato.

That is, green leaves of these potatoes and tomatoes were cut into pieces with widths of 1 - 2 mm, and the resulting pieces were immersed in the above-described enzyme solution POT-9 at 25°C for 16 hours to obtain mesophyll protoplasts.

The thus obtained mesophyll protoplasts were placed in 10 mM aqueous IOA solution at 4°C for 15 minutes and then washed with 0.5 M aqueous mannitol solution 3 times to obtain IOA-treated mesophyll protoplasts.

### Cell Fusion

A cell suspension containing the IOA-treated mesophyll protoplasts at a cell population of 10⁴ - 10⁵ cells/ml and a cell suspension containing the chlorotic mesophyll protoplasts at a cell population of 10⁴ - 10⁵ cells/ml were mixed and the mixed cell suspension was treated with an apparatus for electrofusion (SSH-1 commercially available from Shimazu Corp). To adjust the electroconductivity of the cell suspension, 1 mM aqueous calcium chloride solution was added and the mannitol concentration was decreased from 0.5 M to 0.45 M immediately before the cell fusion operation so as to decrease the osmosis, thereby increasing the percentage of the fused cells. The conditions of the electrofusion are as shown in Table 3 below.

**Table 3**

| Conditions of Electrofusion | |
|---|---|
| Frequency of High Frequency Wave | 1 MHz |
| Voltage of High Frequency Wave | 100 V/cm |
| Time for Applying Voltage | 10 - 20 sec. |
| Pulse (rectangular wave) Voltage | 1.5 KV/cm |
| Pulse Width | 15 µsec. |
| Interval Between Pulses | 0.2 - 1.0 sec. |
| Number of Pulses Applied | 2 - 10 times |

The cell suspension which was subjected to the cell fusion treatment was collected with a Pasteur pipette and was placed in a test tube, followed by centrifugation at an acceleration of 10G for 5 minutes. The supernatant was discarded and the precipitate was suspended in 0.5 M aqueous mannitol solution and the centrifugation was carried out under the same conditions. This washing operation was repeated 3 times and a precipitate was collected. By this operation, almost all of the chlorotic mesophyll protoplasts which were not fused and the fused cells between the chlorotic mesophyll protoplasts were removed together with the supernatant.

### Culturing of Cells Contained in Precipitate

To the thus obtained precipitates, P-1 medium was added so as to attain a cell population of 10⁴ - 10⁵ cells/ml. The composition of P-1 medium is shown in Table 4 below. On the other hand, agarose (SeaPlaque Agarose commercially available from FMC Corporation) was added to P-1 medium to a concentration of 1.6% by weight and the mixture was kept at 40°C so that the agarose is not solidified. Equivolume of the above-mentioned cell suspension and the agarose-containing P-1 medium were mixed and drops thereof were dropped on a petri dish (6 cm diameter) before the agarose is solidified such that the drops are separated each other. The volume of each drop was about 50 - 100 µl and 10 drops were dropped on each petri dish.

The petri dishes were left to stand at a low temperature (3 - 5°C) or at room temperature so as to solidify the agarose. After confirming that the drops were completely solidified, P-1 medium was added to the petri dishes such that the tops of the drops are covered with the medium. The petri dishes were placed in an incubator and the cells were cultured at 25°C in the dark.

**Table 4**

| Composition of Medium for Culturing Protoplasts (P-1 Medium) | |
|---|---|
| Component | Concentration (mg/l) |
| MS Medium* from Which NH₄NO₃ is Removed | |
| myo-Inositol | 1,000.0 |
| 2,4-Dichlorophenoxyacetic Acid (2,4-D) | 0.1 |
| Naphthaleneacetic Acid (NAA) | 0.5 |
| Benzylaminopurine (BA) | 0.5 |
| Glucose | 5,000.0 |
| Sucrose | 10,000.0 |
| Mannitol (pH 5.6) | 46,000.0 |

| | |
|---|---|
| *: Murashige and Skoog Medium (1962) | |

After 30 days' culture, the medium in the vicinities of the drops was removed and the drops were broken such that the cells are not destructed. The cells were dispersed and C-1 medium which is a callus growth medium was added. The cells are then cultured at 25^{o}C in an incubator whose inside is illuminated for 24 hours a day (5,000 lux). The composition of C-1 medium is shown in Table 5 below.

**Table 5**

| Composition of Colony Growth Medium (C-1 Medium) | |
|---|---|
| Component | Concentration (mg/l) |
| MS Medium* from Which NH₄NO₃ is Removed | |
| NH₄NO₃ | 300.0 |
| Naphthaleneacetic Acid (NAA) | 0.1 |
| Benzylaminopurine (BA) | 0.5 |
| Sucrose | 3,000.0 |
| Mannitol (pH 5.6) | 50,000.0 |

| | |
|---|---|
| *: Murashige and Skoog Medium (1962) | |

The calli grown in the callus growth medium to sizes of 1 - 5 mm were transferred to S-1 medium which is a regeneration medium, and the calli were cultured at 20°C in an incubator whose inside is illuminated (5,000 lux) for 16 hours a day, thereby obtaining regenerated plants. The composition of S-1 medium is shown in Table 6 below.

**Table 6**

| Regeneration Medium (S-1 Medium) | |
|---|---|
| Component | Concentration (mg/l) |
| MS Medium* from Which NH₄NO₃ is Removed | |
| NH₄NO₃ | 300.0 |
| Indoleacetic Acid (IAA) | 0.3 |
| Zeatin | 2.0 |
| Coconut Water** | 2.0(%) |
| Sucrose | 3,000.0 |
| Mannitol | 35,000.0 |
| Gellan Gum (Gelrite) (pH 5.8) | 2,000.0 |

| | |
|---|---|
| *: Murashige and Skoog Medium (1962) | |
| **: commercially available from GIBCO | |

The number of the regenerated plants and the number of hybrid plants thus obtained by the method of the present invention are shown in Table 7 below.

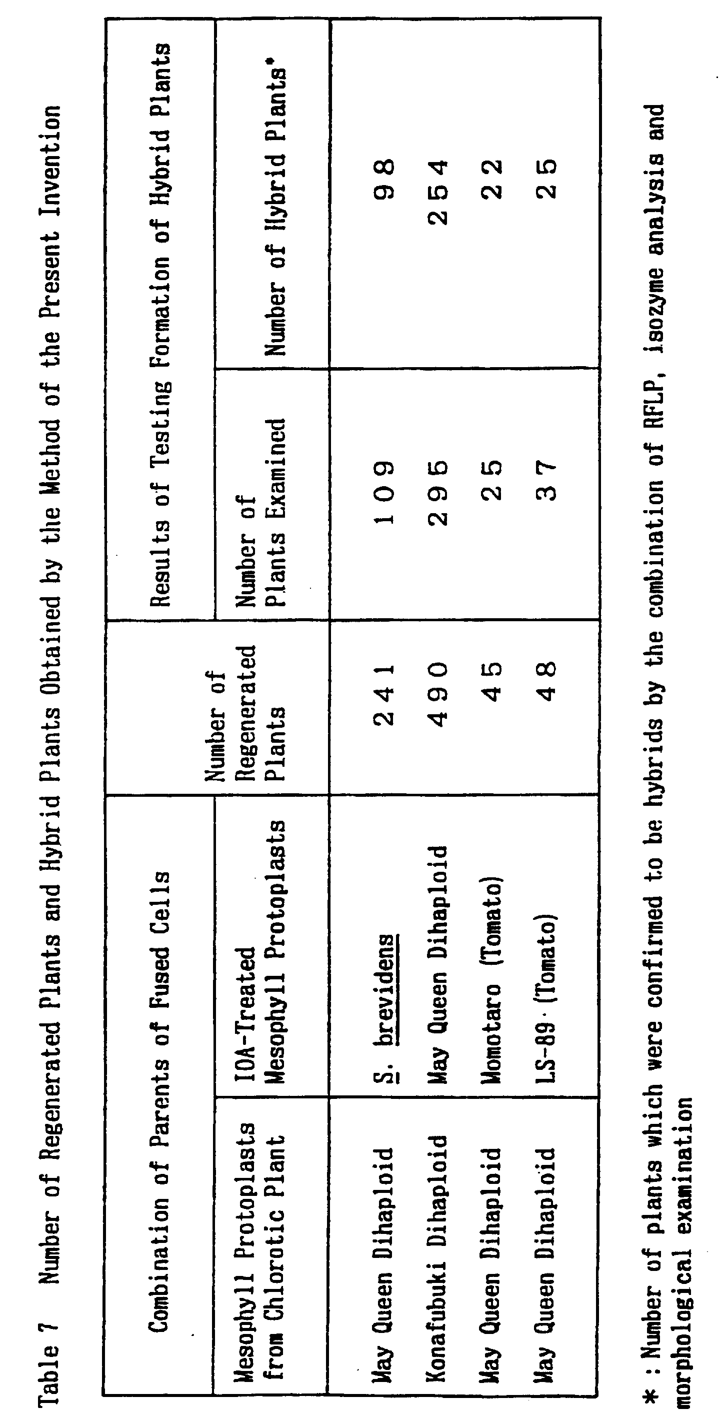

As can be seen from Table 7, by culturing the cells contained in the precipitates, a large number of plants were regenerated. This indicates that the cell culturing in the agarose drops is excellent for regenerating plants. Further, as can be seen from the results showing the number of the hybrids, which was determined by employing a combination of RFLP (restriction fragment length polymorphism) method, isozyme analysis method and examination of morphology, the selection of the desired fused hybrid cells after the cell fusion treatment has high selectivity.

## Claims

1. A method for separating and culturing fused hybrid cells comprising, in the order mentioned, the steps of:
mixing mesophyll protoplasts which cannot divide unless they are fused with a cell having an ability to undergo cell division and chlorotic mesophyll protoplasts originating from a plant treated with norflurazon, and subjecting the mixture to cell fusion treatment;
centrifuging obtained cell suspension; and
culturing cells contained in fraction precipitated by the centrifugation.

2. The method of claim 1, wherein said mesophyll protoplasts which cannot divide unless they are fused with a cell having an ability to undergo cell division are prepared by treating mesophyll protoplasts with a cell-inactivating agent.

3. The method of claim 2, wherein said cell-inactivating agent is selected from the group consisting of iodoacetamide, iodoacetate and diethylpyrrocarbonate.

4. The method of claim 3, wherein said cell-inactivating agent is iodoacetamide.

5. The method of claim 1, wherein said plant treated with norflurazon is a plant whose upper portion alone is chlorotic, which plant is obtained by first culturing seedling of the plant in the absence of norflurazon and then culturing the seedling in the presence of norflurazon, and wherein said chlorotic mesophyll protoplasts are taken from the chlorotic upper portion of the plant.

6. The method of claim 1, wherein said cells contained in the fraction precipitated by the centrifugation are embedded in agarose gel and cultured therein.

7. The method of claim 6, wherein the culturing of said cells is carried out in agarose gel drops each having a volume of 5 - 100 µl.

8. The method of claim 1, wherein said centrifugation is carried out under an acceleration of 7 - 15G for 3 - 5 minutes.

## Patentansprüche

1. Verfahren zur Auftrennung und Kultivierung fusionierter Hybridzellen mit den nachfolgenden Schritten in der angegebenen Reihenfolge:
- Vermischen von Mesophyll-Protoplasten, die sich nicht teilen können, bis sie mit einer Zelle fusioniert werden, die die Fähigkeit zur Zellteilung aufweist, und chlorotischen Mesophyll-Protoplasten, die aus einer mit Norflurazon behandelten Pflanze stammen, und Behandlung der Mischung, um eine Zellfusion zu erreichen;
- Zentrifugieren der so erhaltenen Zellsuspension;
- Kultivieren der Zellen, die in der durch die Zentrifugation abgeschiedenen Fraktion enthalten sind.

2. Verfahren nach Anspruch 1, wobei die Mesophyll-Protoplasten, die sich nicht teilen können, bis sie mit einer Zelle fusioniert werden, die zur Zellteilung befähigt ist, dadurch hergestellt werden, daß die Mesophyll-Protoplasten mit einem zell-inaktivierenden Mittel behandelt werden.

3. Verfahren nach Anspruch 2, wobei das die Zelle inaktivierende Mittel aus der Gruppe ausgewählt wird, bestehend aus Iodacetamid, Iodacetat und Diethylpyrrocarbonat.

4. Verfahren nach Anspruch 3, wobei das die Zelle inaktivierende Mittel Iodacetamid ist.

5. Verfahren nach Anspruch 1, wobei die mit Norflurazon behandelte Pflanze eine Pflanze ist, bei der nur der obere Teil chlorotisch ist, und die Pflanze dadurch erhalten wird, daß zunächst der Keimling der Pflanze in Abwesenheit von Norflurazon kultiviert wird und dann der Keimling in Gegenwart von Norflurazon kultiviert wird, und wobei die chlorotischen Mesophyll-Protoplasten vom chlorotischen oberen Teil der Pflanze genommen werden.

6. Verfahren nach Anspruch 1, wobei die in der durch Zentrifugation abgeschiedenen Fraktion enthaltenen Zellen in ein Agarosegel eingebettet und darin kultiviert werden.

7. Verfahren nach Anspruch 6, wobei die Kultivierung dieser Zellen in Agarosegeltropfen durchgeführt wird, die Je ein Volumen von 5 bis 100 µl aufweisen.

8. Verfahren nach Anspruch 1, wobei die Zentrifugation bei einer Beschleunigung von 7 bis 15 G 3 bis 5 Minuten lang durchgeführt wird.

## Revendications

1. Procédé pour la séparation et la culture de cellules hybrides fusionnées comprenant, dans l'ordre indiqué, les étapes suivantes consistant à :
mélanger des protoplastes du mésophylle qui ne peuvent se diviser à moins d'être fusionnés avec une cellule capable de division cellulaire et des protoplastes du mésophylle chlorotique issus d'une plante traitée par du norfluzaron, et soumettre le mélange à un traitement de fusion cellulaire;
centrifuger la suspension cellulaire obtenue;
cultiver les cellules contenues dans la fraction précipitée par centrifugation.

2. Procédé selon la revendication 1 dans lequel les protoplastes du mésophylle qui ne peuvent se diviser à moins d'être fusionnés avec une cellule capable de division cellulaire sont préparés en traitant les protoplastes du mésophylle avec un agent de désactivation cellulaire.

3. Procédé selon la revendication 2 dans lequel ledit agent de désactivation cellulaire est choisi dans le groupe constitué de l'iodoacétamide, l'iodoacétate et le diéthylpyrrocarbonate.

4. Procédé selon la revendication 3 dans lequel ledit agent de désactivation cellulaire est l'iodoacétamide.

5. Procédé selon la revendication 1 dans lequel ladite plante traitée par le norflurazon est une plante dont seule la partie supérieure est chlorotique, laquelle plante est. obtenue en cultivant d'abord un semis de la plante en l'absence de norflurazon et puis en cultivant le semis en présence de norflurazon, et dans lequel lesdits protoplastes du mésophylle chlorotique sont extraits de la partie supérieure chlorotique de la plante.

6. Procédé selon la revendication 1 dans lequel lesdites cellules contenues dans la fraction précipitée par centrigufation sont introduites dans un lit de gel d'agarose et y sont cultivés.

7. Procédé selon la revendication 6 dans lequel la culture desdites cellules est réalisée dans des gouttes de gel d'agarose, chaque goutte ayant un volume de 5-100 *µ*l.

8. Procédé selon la revendication 1 dans lequel ladite centrifugation est réalisée à une valeur de l'accélération de 7-15 G pendant 3 à 5 minutes.
